# EUROPEAN PATENT SPECIFICATION

(11) **EP 1 471 936 B1**
(45) Date of publication and mention of the grant of the patent: **24.02.2010**
(21) Application number: 03729676.1
(22) Date of filing: 14.01.2003
(51) Int. Cl.: A61K 39/21

(54) **HIV VACCINE AND METHOD OF USE**
HIV-VAKZINE UND ANWENDUNGSVERFAHREN
VACCIN CONTRE LE VIH ET PROCEDE D'UTILISATION

(30) Priority: 14.01.2002 US 348695 P
(43) Date of publication of application: 03.11.2004
(73) Proprietor: Novartis Vaccines and Diagnostics, Inc., Emeryville, CA 94608 (US)
(72) Inventor: VAJDY, Michael, Orinda, CA 94563 (US); SRIVASTAVA, Indresh, Benicia, CA 94510 (US); BARNETT, Susan, San Francisco, CA 94127 (US); O'HAGAN, Derek, Berkeley, CA 94705 (US)
(74) Representative: Marshall, Cameron John
(86) International application number: PCT/US2003/001261
(87) International publication number: WO 2003/059385

(56) References cited:
- WO-A-98/42375
- WO-A1-01/37869
- VAJDY M. ET AL.: "Induction of anti-HIV humoral and cell-mediated immune responses by mucosal immunizations using adjuvants and virus-like particles" CONF. RETROVIRUSES OPPORTUNISTIC INFECT., 30 January 2000 (2000-01-30), XP002383567 page 7:95, abstract 108
- SINGH M ET AL: "Mucosal immunization with HIV-1 gag DNA on cationic microparticles prolongs gene expression and enhances local and systemic immunity" VACCINE, BUTTERWORTH SCIENTIFIC. GUILDFORD, GB, vol. 20, no. 3-4, 12 November 2001 (2001-11-12), pages 594-602, XP004310169 ISSN: 0264-410X
- MCCLUSKIE M J ET AL: "Mucosal immunization of mice using CpG DNA and/or mutants of the heat-labile enterotoxin of Escherichia coli as adjuvants" VACCINE, BUTTERWORTH SCIENTIFIC. GUILDFORD, GB, vol. 19, no. 27, 14 June 2001 (2001-06-14), pages 3759-3768, XP004241795 ISSN: 0264-410X
- DE HAAN L ET AL: "Mucosal immunogenicity of the Escherichia coli heat-labile enterotoxin: role of the A subunit" VACCINE, BUTTERWORTH SCIENTIFIC. GUILDFORD, GB, vol. 14, no. 4, March 1996 (1996-03), pages 260-266, XP004057320 ISSN: 0264-410X
- VERWEIJ W R ET AL: "Musosal immunoadjuvant activity of recombinant Escherichia coli heat-labile enterotoxin and its B subunit: Induction of systemic IgG and secretory IgA responses in mice by intranasal immunization with influenza virus surface antigen" VACCINE, BUTTERWORTH SCIENTIFIC. GUILDFORD, GB, vol. 16, no. 20, December 1998 (1998-12), pages 2069-2076, XP004138458 ISSN: 0264-410X
- PIZZA ET AL.: 'Mucosal vaccines: non toxic derivatives of LT and CT as mucosal adjuvants' VACCINE vol. 19, no. 17-19, 21 March 2001, pages 2534 - 2541, XP004231077
- PIZZA ET AL.: 'LTK63 and LTR72, two mucosal adjuvants ready for clinical trials' INTERNATIONAL JOURNAL OF MEDICAL MICROBIOLOGY vol. 290, no. 4-5, October 2000, pages 455 - 461, XP002967367
- NEIDLEMAN ET AL.: 'Genetically detoxified mutants of heat-labile enterotoxin from escherichia coli are effective adjuvants for induction of cytotoxic T-cell responses against HIV-1 gag-p55' IMMUNOLOGY vol. 101, no. 1, September 2000, pages 154 - 160, XP002967368
- PARTIDOS ET AL.: 'The adjuvant effect of a non-toxic mutant of heat-labile enterotoxin of escherichia coli for the induction of measles virus-specific CTL responses after intranasal co-immunization with a synthetic peptide' IMMUNOLOGY vol. 89, 1996, pages 483 - 487, XP002096389
- COHEN: 'ADIS vaccines show promise after years of frustration' SCIENCE vol. 291, 02 March 2001, pages 1686 - 1688, XP002967369
- SPALDING: 'In hot pursuit of an HIV' VACCINE vol. 10, 1992, pages 24 - 29, XP002967370
- SRIVASTAVA I. ET AL.: "Dynamics of acute and memory mucosal and systemic immune responses against HIV-1 envelope following immunizations through single or combinations of mucosal and systemic routes." VACCINE, vol. 26, 2008, pages 2796-2806,

## Description

### FIELD OF THE INVENTION

The invention relates combination HIV vaccines, specifically for mucosal administration in primates.

### STATE OF THE ART

Acquired immune deficiency syndrome (AIDS) is recognized as one of the greatest health threats facing modes medicine and worldwide sexual transmission of HIV is the leading cause of AIDS. There are, as yet, no cures or vaccines for AIDS. Therefore, construction of a vaccine or drug that can specifically protect against sexual transmission at the site of entry is highly desirable.

In 1983-1984, three groups independently identified the suspected etiological agent of AIDS. *See, e.g.,* Barre-Sinoussi et al. (1983) Science 220:868-871; Montagnier et al., in Human T-Cell Leukemia Viruses (Gallo, Essex & Gross, eds., 1984); Vilmer et al. (1984) The Lancet 1:753; Popovic et al. (1984) Science 224:497-500; Levy et al. (1984) Science 225:840-842. These isolates were variously called lymphadenopathy-associated virus (LAV), human T-cell lymphotropic virus type III (HTLV-III), or AIDS-associated retro virus (ARV). All of these isolates are strains of the same virus, and were later collectively named Human Immunodeficiency Virus (HIV). With the isolation of a related AIDS-causing virus, the strains originally called HTV are now termed HIV-1 and the related virus is called HIV-2 See, e.g., Guyader et al. (1987) Nature 326:662-669; Brun-Vezinet et al. (1986) Science 233:343-346; Clavel et al. (1986) Nature 324:691-695. Consequently, there is a need in the art for compositions and methods suitable for treating and/or preventing HIV infection worldwide.

Although there is some discrepancy as to the effectiveness of cell-mediated or antibody-mediated responses in protection against disease, there is general consensus that generation of both cell-mediated and antibody-mediated responses is highly desirable. Antibody mediated responses would inhibit binding of the virus to its targets in vaginal or rectal tissues, *i.e*., at the site of transmission, whereas cell-mediated responses would play a role in the eradication of infected cells.

Thus, as most HIV infections are transmitted through the female genital tract followed by systemic spread of the virus, induction of local as well as systemic immunity is greatly sought.

Experiments in mice have suggested that mucosal immunization is possible with a combination of Ogp140 and gp120 proteins together with an E.coli heat-labile toxin (Vajdy, Conf. Retroviruses Opportunistic Infect., page 7:95, abstract 108; 30 January 2000). The goal of the present invention is to provide a composition for mucosal administration in primates.

### BRIEF SUMMARY OF THE INVENTION

This invention is directed to pharmaceutical compositions comprising an HIV antigen and a mucosal adjuvant in doses suitable for raising an immune response in a primate by administering them through a mucosal route, preferably intra-nasal, intra-vaginal or intra-rectal. These compositions can be used to treat or prevent HIV infection.

The invention is also directed to the use of an HIV antigen and a mucosal adjuvant in doses suitable for raising an immune response in a primate, for the manifacture of a medicament for administration to primates through a mucosal route, preferably intra-nasal, intra-vaginal or intra-rectal.

The HIV antigens suitable for use in this invention are envelope proteins succh as gp120 and gp160, and antigenic fragments and derivatives thereof, such as oligomeric gp140 (Ogp140). Preferably, the antigens are optimized for immunogenicity. The mucosal adjuvants suitable for use in this inventions are detoxified mutants of E. coli heat labile toxin (LT) such as LTR72 and LTK63.

### DETAILED DESCRIPTION OF THE INVENTION

### 1. Definitions

In order to facilitate an understanding of the invention, selected terms used in the application will be discussed below.

The term "polynucleotide", as known in the art, generally refers to a nucleic acid molecule. A "polynucleotide" can include both double- and single-stranded sequences and refers to, but is not limited to, cDNA from viral, prokaryotic or eukaryotic MRNA, genomic RNA and DNA sequences from viral (e.g. RNA and DNA viruses and retroviruses) or prokaryotic DNA, and especially synthetic DNA sequences. The term also captures sequences that include any of the known base analogs of DNA and RNA, and includes modifications such as deletions, additions and substitutions (generally conservative in nature), to the native sequence, so long as the nucleic acid molecule encodes a therapeutic or antigenic protein. These modifications may be deliberate, as through site-directed mutagenesis, or may be accidental, such as through mutations of hosts that produce the antigens. Modifications of polynucleotides may have any number of effects including, for example, facilitating expression of the polypeptide product in a host cell.

The polynucleotides used in the present invention include polynucleotides encoding for an immunogenic fragment or derivative thereof. Such immunogenic fragments or derivatives thereof include fragments encoding for a B-cell epitope or a T-cell epitope as discussed below.

As used herein, the terms "polypeptide" and "protein" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include postexpression modifications of the polypeptide, for example, glycosylation, acetylation, phosphorylation and the like. Furthermore, for purposes of the present invention, a "polypeptide" refers to a protein that includes modifications, such as deletions, additions and substitutions (generally conservative in nature), to the native sequence, so long as the protein maintains the desired activity. These modifications may be deliberate, as through site-directed mutagenesis, or may be accidental, such as through mutations of hosts that produce the proteins or errors due to PCR amplification.

By "" is meant, when referring to a polynucleotide or a polypeptide, that the indicated molecule is separate and discrete from the whole organism with which the molecule is found in nature or, when the polynucleotide or polypeptide is not found in nature, is sufficiently free of other biological macromolecules so that the polynucleotide or polypeptide can be used for its intended purpose.

The phrase "antigen", as used herein, refers to a molecule containing one or more epitopes (either linear, conformational or both) that will stimulate a host's immune system to make a humoral and/or cellular antigen-specific response. The term is used interchangeably with the term "immunogen." Normally, a B-cell epitope will include at least about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes.

Furthermore, for purposes of the present invention, an "antigen" refers to a protein that includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts that produce the antigens.

An "immunological response" to an antigen or composition is the development in a subject of a humoral and/or a cellular immune response to an antigen present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, specific effector cells, such as B and plasma cells as well as cytotoxic T cells, against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells. In addition, a chemokine response may be induced by various white blood or endothelial cells in response to an administered antigen.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, e.g., Erickson et al., J. Immunol. (1993) 151:4189-4199; Doe et al., Eur. J. Immunol. (1994) 24:2369-2376. Recent methods of measuring cell-mediated immune response include measurement of intracellular cytokines or cytokine secretion by T-cell populations (*e.g*., by ELISPOT technique), or by measurement of epitope specific T-cells (*e.g*., by the tetramer technique)(reviewed by McMichael, A.J., and O'Callaghan, C.A., J. Exp. Med. 187(9):1367-1371, 1998; Mcheyzer-Williams, M.G., et al, Immunol. Rev. 150:5-21, 1996; Lalvani, A., et al, J. Exp. Med. 186:859-865, 1997).

Thus, an immunological response as used herein may be one that stimulates the production of CTLs, and/or the production or activation of helper T- cells. The production of chemokines and/or cytokines may also be stimulated. The antigen of interest may also elicit an antibody-mediated immune response. Hence, an immunological response may include one or more of the following effects: the production of antibodies by B-cells; and/or the activation of suppressor, cytotoxic, or helper T-cells and/or T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

### 2. Pharmaceutical Compositions.

The compositions of the inventions are HIV vaccines to be administered through a mucosal route, included but not limited to oral, intranasal, intragastric, pulmonary, intestinal, rectal, ocular and vaginal. A preferred mucosal route is intranasal.

Where the mucosal delivery is by an intranasal route, the vaccine of the invention may be in the form of a nasal spray, nasal drops, gel or powder.

Where it is for oral route, for instance, it may be in the form of tablets of capsules (optionally enteric-coated), liquid, transgenic plants, etc. The compositions of the invention thus comprise HIV envelope antigens and mucosal adjuvants.

### a) Antigens

The antigens used in this invention are derived from an HIV env protein, such as gp140. Preferably, the antigens of the invention are optimized for immunogenicity and oligomerized, such as Ogp140. A dose suitable for administration in primates is 300 µg of env protein antigen.

The genes of HIV are located in the central region of the proviral DNA and encode at least nine proteins divided into three major classes: (1) the major structural proteins, Gag, Pol, and Env; (2) the regulatory proteins, Tat and Rev and (3) the accessory proteins, Vpu, Vpr, Vif, and Nef. Many variants are known in the art, including HIV_{SF2}, HIV_{IIIb}, HIV_{SF2}, HIV-1_{SF162}, HN-1_{SF170}, HIV_{LAV}, HIV_{LAI}, HIV_{MN}, HIV-1_{CM235}, HIV-1_{US4}, other HIV-1 strains from diverse subtypes (e.g., subtypes, A through G, and O), HIV-2 strains and diverse subtypes (e.g., HIV-2_{UC1} and HIV-2_{UC2}), and simian immunodeficiency virus (SIV). (See, e.g., Virology, 3rd Edition (W.K. Joklik ed. 1988); Fundamental Virology, 2nd Edition (B.N. Fields and D.M. Knipe, eds. 1991); Virology, 3rd Edition (Fields, BN, DM Knipe, PM Howley, Editors, 1996, Lippincott-Raven, Philadelphia, PA; for a description of these and other related viruses).

In addition, due to the large immunological variability that is found in different geographic regions for the open reading frame of HIV, particular combinations of antigens may be preferred for administration in particular geographic regions. Briefly, at least eight different subtypes of HIV have been identified and, of these, subtype B viruses are more prevalent in North America, Latin America and the Caribbean, Europe, Japan and Australia. Almost every subtype is present in sub-Saharan Africa, with subtypes A and D predominating in central and eastern Africa, and subtype C in southern Africa. Subtype C is also prevalent in India and it has been recently identified in southern Brazil. Subtype E was initially identified in Thailand, and is also present in the Central African Republic. Subtype F was initially described in Brazil and in Romania. The most recent subtypes described are G, found in Russia and Gabon, and subtype H, found in Zaire and in Cameroon. Group O viruses have been identified in Cameroon and also in Gabon. Thus, as will be evident to one of ordinary skill in the art, it is generally preferred to select an HIV antigen that is appropriate to the particular HIV subtype that is prevalent in the geographical region of administration. Subtypes of a particular region may be determined by two-dimensional double immunodiffusion or, by sequencing the HIV genome (or fragments thereof) isolated from individuals within that region. Importantly, we have found that antibodies induced by immunizations with Ogp140 can neutralize various strains of HIV and therefore can be used as a prophylactic vaccine in several regions of the world.

As described above, also presented by HIV are various *Gag* and *Env* antigens. HIV-1 *Gag* proteins are involved in many stages of the life cycle of the virus including, assembly, virion maturation after particle release, and early post-entry steps in virus replication. The roles of HIV-1 *Gag* proteins are numerous and complex (Freed, E.O. (1998) Virology 251:1-15). For its part, the envelope protein of HIV-1 is a glycoprotein of about 160 kD (gp160). During virus infection of the host cell, gp160 is cleaved by host cell proteases to form gp120 and the integral membrane protein, gp41. The gp41 portion is anchored in (and spans) the membrane bilayer of virion, while the gp 120 segment protrudes into the surrounding environment. As there is no covalent attachment between gp120 and gp41, free gp120 is released from the surface of virions and infected cells.

The sequences encoding the open reading frame of the ectodomain of the Env protein (gp140) from the HIV-1_{US4} strain were codon-optimized as described elsewhere [Haas, 1996 #562; zur Megede, 2000 #1451], and constructed synthetically as a 2.1 kb EcoR1-Xba1 DNA fragment (Midland Reagent Company, Midland, TX). This gene cassette contained the protein-encoding region of the Env protein fused in frame to the human tissue plasminogen activator (tPA) signal sequence as previously described [Chapman, 1991 #1550]. In order to stabilize the oligomeric structure of the encoded gp140 protein, the DNA sequence was mutated to introduce an arginine to serine change in the primary protease cleavage site (REKR) in the Env polypeptide [Earl, 1990 #2906](Fig.1A). The resulting Env expression cassette (gp140) was cloned into the EcoR1-Xba1 sites of the pCMV3 expression vector for the derivation of stable CHO cell lines. This vector contains the CMV enhancer/promoter elements, an ampicillin resistance gene, and sequences encoding a fusion protein composed of dihydrofolate reductase (DHFR) and an attenuated neomycin resistance protein.

At least one immunogenic portion of an HIV antigen may be used for mucosal immunization. As utilized herein, "immunogenic portion" refers to a portion of the respective antigen that is capable, under the appropriate conditions, of causing an immune response (i.e., cell-mediated or humoral). The immunogenic portion(s) used for immunization may be of varying length, although it is generally preferred that the portions be at least 9 amino acids long and may include the entire antigen. Immunogenicity of a particular sequence is often difficult to predict, although T cell epitopes may be predicted utilising computer algorithms such as TSITES (MedImmune, Maryland), in order to scan coding regions for potential T-helper sites and CTL sites. From this analysis, peptides are synthesized and used as targets in an *in vitro* cytotoxic assay. Other assays, however, may also be utilized, including, for example, ELISA, or ELISPOT, which detects the presence of antibodies against the newly introduced vector, as well as assays which test for T helper cells, such as gamma-interferon assays, IL-2 production assays and proliferation assays.

Immunogenic portions may also be selected by other methods. For example, the HLA A2.1 transgenic mouse has been shown to be useful as a model for human T-cell recognition of viral antigens. Briefly, in the influenza and hepatitis B viral systems, the murine T cell receptor repertoire recognizes the same antigenic determinants recognized by human T cells. In both systems, the CTL response generated in the HLA A2.1 transgenic mouse is directed toward virtually the same epitope as those recognized by human CTLs of the HLA A2.1 haplotype (Vitiello et al. (1991) J. Exp. Med. 173:1007-1015; Vitiello et al. (1992) Abstract of Molecular Biology of Hepatitis B Virus Symposia).

Additional immunogenic portions of the HIV antigens described herein may be obtained by truncating the coding sequence at various locations including, for example, to include one or more epitopes from the various domains of the HIV genome. As noted above, such domains include structural domains such as *Gag, Gag-polymerase, Gag-protease, reverse transcriptase (RT), integrase (IN)* and *Env.* The structural domains are often further subdivided into polypeptides, for example, p55, p24, p6 (*Gag*)*;* p160, p10, p15, p31, p65 (*pol, prot, RT and IN);* and gp160, gp120 and gp41 (*Env*) or Ogp140 as constructed by Chiron Corporation. Additional epitopes of HIV and other sexually transmitted diseases are known or can be readily determined using methods known in the art.

Preferably, the antigens of this invention are optimized for immunogenicity, such as Ogp140.

As used herein, the phrase "optimized" refers to an increase in the immunogenicity of the proteins, so that they can induce higher quantity and quality of antibodies. Moreover, polynucleotide sequences that can encode Ogp140 can be optimized by codon substitution of wild type sequences. Haas, et al., (Current Biology 6(3):315-324, 1996) suggested that selective codon usage by HIV-1 appeared to account for a substantial fraction of the inefficiency of viral protein synthesis. Andre, et al., (J. Virol. 72(2):1497-1503, 1998) described an increased immune response elicited by DNA vaccination employing a synthetic gp120 sequence with optimized codon usage. Schneider, et al. (J. Virol. 71 (7):4892-4903, 1997) discuss inactivation of inhibitory (or instability) elements (INS) located within the coding sequences of the Gag and Gag-protease coding sequences.

The sequences encoding codon-optimized gp140 were cloned into an expression vector for the evaluation of Env expression in transient transfection experiments and for protein purification. To facilitate the efficient secretion of recombinant Ogp140 protein, the native HIV signal sequence was replaced by the human tissue-type plasminogen activator (t-PA) signal sequence. The effect of codon optimization on gp140 expression was determined by transient transfection of 293 cells with codon-optimized and native (non-codon optimized) gp140 constructs and, comparison of expression levels by a capture ELISA and immunoblotting. It was shown previously that sequence modification of HIV gag dramatically improved the level of expression [zur Megede, 2000 #1451], similarly, codon optimization also improved the expression of gp140 4 to 10 fold compared to the native construct [Haas, 1996 #562]. Using such sequence-modified constructs we developed stable CHO cell lines secreting 5-15 µg/ml of o-gp140 and gp120. The antigenicity of oligomeric gp140 with and without a point mutation (R509 to S509) in the gp120/g41 primary protease cleavage site was also evaluated by transiently transfecting the 293 cells. Expression and structural characterization data indicated that the native form of the HIV-1 ectodomain-encoding region did not form gp140 oligomers efficiently (only about 50% of the expressed protein was found to be in oligomeric conformation). In contrast, the single R to S mutation in the protease cleavage site resulted in the expression of stable gp140 protein in its oligomeric conformation. Therefore, the constructs employing the protease cleavage site mutation were used for the derivation of stable CHO cell lines for protein production. Cell lines were also derived for the monomeric US4 gp120. Expression for these stable CHO cell lines ranged from 1-15 µg/ml of secreted Env glycoprotein.

The antigens in the immunogenic compositions of the invention are HIV env proteins. The proteins can be prepared by various means (e.g., native expression, recombinant expression, purification from cell culture, chemical synthesis etc.) and in various forms (e.g., native, fusions etc.). They are preferably prepared in substantially pure form (i.e., substantially free from other bacterial or host cell proteins.

### b) Mucosal adjuvants

The compositions of the invention also include a mucosal adjuvant suitable for mucosal delivery, preferably intra-nasal, intra-vaginal or intra-rectal delivery. The mucosal adjuvant for use in the invention are detoxified E. coli heat-labile enterotoxins (LT), i.e., LT that comprise a mutated A subunit and a B subunit, wherein the mutation in the A subunit changes the serine of position 63 and/or the alanine of position 72. Preferred examples thereof are the K63 or the R72 mutants. A dose of detoxified LT mucosal adjuvant suitable for administration in primates is of 100 µg.

*E. coli* heat-labile toxins are generally ADP-ribosylating bacterial toxins. These toxins are composed of a monomeric, enymatically active A subunit which is responsible for ADP-ribosylation of GTP-binding proteins, and a non-toxic B subunit which binds receptors on the surface of the target cell and delivers the A subunit across the cell membrane. The A subunit of wildtype LT is known to increase intracellular cAMP levels in target cells, which the B subunit is pentameric and is thought to bind to GM1 ganglioside receptors. (LT-B is also thought to bind to additional receptors).

Generally, the wildtype ADP-ribosylating toxins are too toxic for use in humans. One approach to eliminate or decrease the toxicity of these proteins is to mutate one or more amino acids in the A subunit. Detoxified ADP-ribosylating toxin mutants are known in the art, including LTK63 and LTR72. See, e.g., WO 98/42375, WO98/18928, and WO 97/02348.

As used herein, "detoxified" refers to both completely nontoxic and low residual toxic mutants of the toxin in question. Preferably, the detoxified protein retains a toxicity of less than 0.01% of the naturally occurring toxin counterpart, more preferably less than 0.001 % and even more preferably, less than 0.0001% of the toxicity of the naturally occurring toxin counterpart. The toxicity may be measured in mouse CHO cells or preferably by evaluation of the morphological changes in T1 cells. In particular, Y1 cells are adrenal tumor epithelial cells which become markedly more rounded when treated with a solution containing LT. (Ysamure et al., Cancer Res. (1966) 26:529 - 535). The toxicity of LT is correlated with this morphological transition. Thus, the mutant toxins may be incubated with Y1 cells and the morphological changes of the cells assessed.

The term "toxoid" as used herein generally refers to a genetically detoxified toxin.

A detoxified mutant of an E. coli heat labile toxin can comprise one or more amino acid additions, deletions or substitutions that result in a molecule having reduced toxicity while retaining adjuvanticity, like the mutants used in the present invention and described above. If an amino acid is substituted for the wild-type amino acid, such substitutions may be with a naturally occurring amino acid or may be with a modified or synthetic amino acid. Substitutions which alter the amphotericity and hydrophilicity while retaining the steric effect of the substituting amino acid as far as possible are generally preferred.

The mutants used in the compositions and methods of the invention are in the form of a holotoxin, comprising the mutated A subunit and the B subunit, which may be oligomeric, as in the wild-type holotoxin. The B subunit is preferably not mutated.

Preferred LT mutants for use in the methods and compositions of the invention include mutants with one or more of the following mutations: a mutation in the A subunit of the serine at position 63, and a mutation in the A subunit of the alanine at position 72, both numbered relative to the Domenighini reference discussed below. Preferably, the serine at position 63 is replaced with a lysine and the alanine at position 72 is replaced with arginine.
Preferably, the mucosal adjuvants of the invention are LT mutants R72 and K63.

For purposes of the present invention, the numbering of LT corresponds to the LT sequences set forth in Domenighini et al., Molecular Microbiol. (1995) 15:1165 - 1167. This Domenighini reference is incorporated by reference in its entirety in this application. Specifically, the LT sequences set forth and described in this Domenighini reference are specifically incorporated herein by reference in their entirety.

### c) Other components

Microparticles can also be used with the invention as mucosal adjuvants. These are preferably derived from a poly(a-hydroxy acid), in particular, from a poly(lactide) ("PLA"), a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials which have a variety of molecular weights and, in the case of the copolymers such as PLG, a variety of lactide:glycolide ratios, the selection of which will be largely a matter of choice, depending in part on the coadministered antigen. The antigen may be entrapped within the microparticles, or may be adsorbed onto their surfact.

One or more HIV antigens can be used with the vaccine and methods of this invention. For instance, Ogp140 antigens can be used with gag antigens. For example, the Ogp140-containing and the gag-containing microparticles may be a mixture of two distinct populations of microparticles, the first containing Ogp140 and the second containing gag. Alternatively, the microparticles maybe present as a single population, with Ogp140 and gag (and any further antigens) distributed evenly.

LT mutants may advantageously be used in combination with microparticle-entrapped antigen, resulting in significantly enhanced immune responses.

Optionally, an immuno-modulatory factor may be added to the pharmaceutical composition.

As used here, an "immuno-modulatory factor" refers to a molecule, for example a protein that is capable of modulating an immune response. Non-limiting examples of immunomodulatory factors include lymphokines (also known as cytokines), such as IL-6, TGF-β, IL-1, IL-2, IL-3, etc.); and chemokines (*e.g*., secreted proteins such as macrophage inhibiting factor). Certain cytokines, for example TRANCE, flt-3L, and a secreted form of CD40L are capable of enhancing the immunostimulatory capacity of APCs. Non-limiting examples of cytokines which may be used alone or in combination with the present invention include, interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-1α), interleukin-11 (IL-11), MIP-1γ, leukemia inhibitory factor (LIF), c-kit ligand, thrombopoietin (TPO), CD40 ligand (CD40L), tumor necrosis factor-related activation-induced cytokine (TRANCE) and flt3 ligand (flt-3L). Cytokines are commercially available from several vendors such as, for example, Genzyme (Framingham, MA), Amgen (Thousand Oaks, CA), R&D Systems and Immunex (Seattle, WA). The sequences of many of these molecules are also available, for example, from the GenBank database. It is intended, although not always explicitly stated; that molecules having similar biological activity as wild-type or purified cytokines (e.g., recombinantly produced or mutants thereof) and nucleic acid encoding these molecules are intended to be used with the invention.

The compositions of the invention will typically be formulated with pharmaceutically acceptable carriers or diluents. As used herein, the term "pharmaceutically acceptable carrier" refers to a carrier for administration of the antigens which does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee et al. (1997) J Microencapsul. 14(2):197-210; O'Hagan et al. (1993) Vaccine 11(2):149-54. Such carriers are well known to those of ordinary skill in the art. Additionally, these carriers may function as immunostimulating agents ("adjuvants"). Furthermore, the antigen may be conjugated to a bacterial toxoid, such as toxoid from diphtheria, tetanus, cholera, etc., as well as toxins derived from *E. coli.*

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of acceptable excipients is available in the well-known *Remington's Pharmaceutical Sciences.*
Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

Further, the compositions described herein can include various excipients, adjuvants, carriers, auxiliary substances, modulating agents, and the like.

Additional adjuvants may also be used with the invention. Such adjuvants include, but are not limited to: (1) cytokines, such as interleukins (IL-1, IL-2, etc.), macrophage colony stimulating factor (M-CSF), tumor necrosis factor (TNF), beta chemokines-(MIP, 1-alpha, 1-beta Rantes, etc.); (2) detoxified mutants of a bacterial ADP-ribosylating toxin such as a cholera toxin (CT), a pertussis toxin (PT), or an *E*. *coli* heat-labile toxin (LT), particularly LT-K63 (where lysine is substituted for the wild-type amino acid at position 63) LT-R72 (where arginine is substituted for the wild-type amino acid at position 72), CT-5109 (where serine is substituted for the wild-type amino acid at position 109), and PT-K9/G129 (where lysine is substituted for the wild-type amino acid at position 9 and glycine substituted at position 129) (see, e.g., International Publication Nos. W093/13202; W092/19265; WO 95/17211; WO 98/18928 and WO 01/22993); and (3) other substances that act as immunostimulating agents to enhance the effectiveness of the composition; oligodeoxy nucleotides containing immunostimulatory CpG motifs (Cpg); or combinations of any of the above.

### 3. Methods

The compositions disclosed herein can be administered to a subject to generate an immune response. Preferably, the composition can be used as a vaccine to treat or prevent HIV infection.

As used herein, "subject" is meant, humans and other primates, including non-human primates such as chimpanzees and other apes and monkey species. The term does not denote a particular age. Thus, both adult and newborn individuals are intended to be covered.

The compositions will include "immunologically effective amounts" of HIV antigen *i.e*. amounts sufficient to raise a specific immune response or, more preferably, to treat, reduce, or prevent HIV infection. An immune response can be detected by looking for antibodies to the HIV antigen used (*e.g*. IgG or IgA) in patient samples (*e.g*. in blood or serum, in mesenteric lymph nodes, in spleen, in gastric mucosa, and/or in faeces).

### 4. Techniques and Further Definitions

The practice of the present invention will employ, unless otherwise indicated, conventional techniques of molecular biology, microbiology, recombinant DNA, and immunology, which are within the skill of the art. Such techniques are explained fully in the literature *eg.* Sambrook Molecular Cloning; A Laboratory Manual, Second Edition (1989); DNA Cloning, Volumes I and II (D.N Glover ed. 1985); Oligonucleotide Synthesis (M.J. Gait ed, 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller & M.P. Calos eds. 1987, Cold Spring Harbor Laboratory); Mayer & Walker, eds. (1987), Immunochemical Methods in Cell and Molecular Biology (Academic Press, London); Scopes, (1987) Protein Purification: Principles and Practice, Second Edition (Springer-Verlag, N.Y.), and Handbook of Experimental Immunology, Volumes I-IV (Weir & Blackwell eds 1986).

The term "comprising" means "including" as well as "consisting", so a composition "comprising" X may consist exclusively of X or may include something additional *e.g*. X + Y.

A composition containing X is "substantially free" from Y when at least 85% by weight of the total X+Y in the composition is X. Preferably, X comprises at least ~90% by weight of the total of X+Y in the composition, more preferably at least ~95% or even 99% by weight

### EXAMPLE

The following example is offered by way of illustration, and not by way of limitation.

This example demonstrates the induction of an immune response in rhesus macaques through mucosal immunization with HIV-1 gag and HIV-1 Ogp140.

Two groups of rhesus macaques were immunized intranasally (IN) with a combination of HIV-1 gag (p24) and HIV-1 Ogp. Each group contained two animals. The animals in Group One were immunized in the presence of LTK63. The animals in Group Two were immunized in the presence of LTR72. The formulations used for each group are set forth below in Table 1.

**Table 1: Immunization Formulations**

| | Ogp140 | gag | LTK63 | LTR72 |
|---|---|---|---|---|
| Group One | 300µg | 300µg | 100µg | -- |
| Group Two | 300µg | 300µg | 100µg | -- |
| Group Three | 300µg | 300µg | -- | 100µg |
| Group Four | 300µg | 300µg | -- | 100µg |

An antibody mediated response was observed after the course of five immunizations. Serum IgG titers for each animal two weeks post the fourth immunization (2wp4) and two weeks post the fifth immunization (2wp5) are set forth in Tables 2 and 3 below. Table 2 contains the anti-Ogp140 antibody titers. Table 3 contains the anti-gag (p24) antibody titers. Vaginal wash IgA titers for each animal are set forth in Tables 4 and 5 below. Table 4 contains the anti-Ogp antibody titers. Table 5 contains the anti-gag (p24) antibody titers.

**Table 2: Serum Anti-Ogp140 IgG Titers**

| Group One | | 2wp4 | 2wp5 |
|---|---|---|---|
| | Animal One | 2,165 | 4,996 |
| | Animal Two | 21,573 | 6,528 |

| Group Two | | | |
|---|---|---|---|
| | Animal One | 464 | 712 |
| | Animal Two | 7,425 | 3,665 |

**Table 3: Serum Anti-gag (p24) IgG Titers**

| Group One | | 2wp4 | 2wp5 |
|---|---|---|---|
| | Animal One | 44 | 1326 |
| | Animal Two | 553 | 813 |

| Group Two | | | |
|---|---|---|---|
| | Animal One | 30 | 164 |
| | Animal Two | 353 | 4877 |

**Table 4: Vaginal Wash Anti-Ogp140 IgA Titers**

| Group One | | 2wp4 | 2wp5 |
|---|---|---|---|
| | Animal One | 1333 | 35 |
| | Animal Two | 154 | 135 |

| Group Two | | | |
|---|---|---|---|
| | Animal One | 95 | 217 |
| | Animal Two | 86 | 335 |

**Table 5: Vaginal Wash Anti-gag (p24) IgA Titers**

| Group One | | 2wp4 | 2wp5 |
|---|---|---|---|
| | Animal One | 16 | 2 |
| | Animal Two | 2.5 | 1.5 |

| Group Two | | | |
|---|---|---|---|
| | Animal One | 17 | 4 |
| | Animal Two | 15.5 | 67 |

## Claims

1. A composition suitable for mucosal delivery to primates, comprising 300µg of an HIV envelope antigen and 100µg of a detoxified mutant holotoxin of *E.coli* heat labile toxin (LT), comprising a mutated A subunit and a B subunit, wherein the mutation in the A subunit is selected from one or more of the group consisting of a mutation of the serine of position 63 and a mutation of the alanine of position 72.

2. The composition of claim 1, wherein said envelope protein is selected from the group consisting of gp 120, gp 160 and ogp 140.

3. The composition of any preceding claim, wherein said HIV envelope antigen is optimised for immunogenicity.

4. The composition of any preceding claim, wherein said composition is suitable for intranasal delivery, intravaginal delivery or intrarectal delivery.

5. The composition of any previous claim, for use as a medicament.

6. The use of 300µg of an HIV envelope antigen and 100µg of a detoxified mutant holotoxin of *E.coli* heat labile toxin (LT), comprising a mutated A subunit and a B subunit, wherein the mutation in the A subunit is selected from one or more of the group consisting of a mutation of the serine of position 63, and a mutation of the alanine of position 72, in the manufacture of a medicament for use in raising an immune response in a primate subject after mucosal administration of said medicament.

7. The use of claim 6, wherein said envelope protein is selected from the group consisting of gp 120, gp 160 and ogp 140.

8. The use of claim 6 wherein said medicament is administered intranasally, intravaginally or intrarectally.

## Patentansprüche

1. Zusammensetzung, die für die mukosale Verabreichung an Primaten geeignet ist, und die 300 µg eines HIV-Hüllantigens und 100 µg eines detoxifizierten, mutierten Holotoxins des hitzelabilen Toxins (LT) von *E. coli* umfasst, welches eine mutierte Untereinheit A und eine mutierte Untereinheit B umfasst, wobei die Mutation in der Untereinheit A ausgewählt ist aus einer oder aus mehreren Mutationen der Gruppe, die aus einer Mutation des Serins in Position 63 und einer Mutation des Alanins in Position 72 besteht.

2. Zusammensetzung nach Anspruch 1, wobei das Hüllprotein ausgewählt ist aus der Gruppe bestehend aus gp120, gp160 und ogp140.

3. Zusammensetzung nach einem der vorhergehenden Ansprüche, wobei das HIV-Hüllantigen hinsichtlich der Immunogenität optimiert wurde.

4. Zusammensetzung nach einem der vorhergehenden Ansprüche, wobei die Zusammensetzung für die intranasale Verabreichung, die intravaginale Verabreichung oder die intrarektale Verabreichung geeignet ist.

5. Zusammensetzung nach einem der vorhergehenden Ansprüche zur Verwendung als Medikament.

6. Verwendung von 300 µg eines HIV-Hüllantigens und 100 µg eines detoxifizierten, mutierten Holotoxins des hitzelabilen Toxins (LT) von *E*. *coli,* welches eine mutierte Untereinheit A und eine mutierte Untereinheit B umfasst, wobei die Mutation in der Untereinheit A ausgewählt ist aus einer oder aus mehreren Mutationen der Gruppe, die aus einer Mutation des Serins in Position 63 und einer Mutation des Alanins in Position 72 besteht, zur Herstellung eines Medikaments zur Verwendung bei der Erzeugung einer Immunantwort in einem Primatensubjekt nach mukosaler Verabreichung des Medikaments.

7. Verwendung nach Anspruch 6, wobei das Hüllprotein ausgewählt ist aus der Gruppe bestehend aus gp120, gp160 und ogp140.

8. Verwendung nach Anspruch 6, wobei das Medikament intransal, intravaginal oder intrarektal verabreicht wird.

## Revendications

1. Composition appropriée pour une délivrance mucosale à des primates comprenant 300 µg d'un antigène de l'enveloppe du VIH et 100 µg d'une holotoxine mutante détoxifiée de la toxine thermolabile (LT) de *E. coli*, comprenant une sous-unité A mutée et une sous-unité B, où la mutation dans la sous-unité A est choisie parmi une ou plusieurs du groupe constitué par une mutation de la sérine en position 63 et une mutation de l'alanine en position 72.

2. Composition selon la revendication 1, dans laquelle ladite protéine de l'enveloppe est choisie dans le groupe constitué par gp120, gp160 et ogp140.

3. Composition selon l'une quelconque des revendications précédentes, dans laquelle ledit antigène de l'enveloppe du VIH est optimisé pour l'immunogénicité.

4. Composition selon l'une quelconque des revendications précédentes, où ladite composition est appropriée pour une délivrance intranasale, une délivrance intravaginale ou une délivrance intrarectale.

5. Composition selon l'une quelconque des revendications précédentes, pour une utilisation en tant que médicament.

6. Utilisation de 300 µg d'un antigène de l'enveloppe du VIH et de 100 µg d'une holotoxine mutante détoxifiée de la toxine thermolabile (LT) de *E*. *coli,* comprenant une sous-unité A mutée et une sous-unité B, où la mutation dans la sous-unité A est choisie parmi une ou plusieurs du groupe constitué par une mutation de la sérine en position 63 et une mutation de l'alanine en position 72, dans la fabrication d'un médicament à utiliser pour soulever une réponse immunitaire chez un sujet primate après l'administration mucosale dudit médicament.

7. Utilisation selon la revendication 6, où ladite protéine de l'enveloppe est choisie dans le groupe constitué par gp120, gp160 et ogp140.

8. Utilisation selon la revendication 6, où ledit médicament est administré par voie intranasale, intravaginale ou intrarectale.
